# EUROPÄISCHE PATENTSCHRIFT

(11) **EP 0 643 575 B1**
(45) Veröffentlichungstag und Bekanntmachung des Hinweises auf die Patenterteilung: **06.03.1996**
(21) Anmeldenummer: 93909781.2
(22) Anmeldetag: 17.05.1993
(51) Int. Cl.: A61K 9/00, A61K 31/135

(54) **DOSIERSPRAY FÜR PERNASALE APPLIKATION**
METERING SPRAY DESIGNED FOR PERNASAL APPLICATION
NEBULISEUR-DOSEUR POUR APPLICATIONS PAR VOIE NASALE

(30) Priorität: 03.06.1992 DE 4218291
(43) Veröffentlichungstag der Anmeldung: 22.03.1995
(73) Patentinhaber: ARROWDEAN LTD., IE-Dublin 2 (IE)
(72) Erfinder: MATTERN, Claudia, D-8130 Starnberg (DE); HÄCKER, Rüdiger, A-6600 Greiz (AT)
(74) Vertreter: Winkler, Andreas, Dr.
(86) Internationale Anmeldenummer: DE9300442
(87) Internationale Veröffentlichungsnummer: WO9324107

(56) Entgegenhaltungen:
- EP-A- 0 160 501
- FR-M- 6 033

## Beschreibung

Die Erfindung betrifft die neuartige Verwendung eines Dosiersprays für pernasale Applikation.

Eine Vielzahl von pharmazeutischen Wirkstoffen entfalten ihre eigentliche pharmakodynamische Wirkung im zentralen Nervensystem. So haben neueste Untersuchungen ergeben, daß Sexualhormone und ihre Derivate teilweise unerwartete positive Seitenwirkungen im zentralen Nervensystem zeigen, insbesondere indem die allgemeine psychophysiologische Belastbarkeit des Patienten merkbar ansteigt. Andere Wirkstoffe, wie bestimmte biogene Amine hängen in ihrer therapeutischen Wirksamkeit direkt von einem erleichterten Zutritt zum zentralen Nervensystem ab. So ist dieser bspw. für Medikamente zur Behandlung der Parkinsonschen Krankheit, wie bspw. Dopamin, Dopamin-Derivate, NADH oder NADPH, eine entscheidende Wirkungsvoraussetzung.

Werden Wirkstoffe aufgrund ihrer chemischen Eigenschaften im Magen-Darm-Kanal durch die wechselnden pH-Verhältnisse und die enzymatischen Vorgänge verändert (wie bspw. die Mehrzahl der biogenen Amine) oder sind sie nur eingeschränkt wasserlöslich (wie die Sexualhormone), sind bisher aufwendige galenische Darreichungsformen erforderlich, oder es muß auf die an den Arzt gebundene und belastende parenterale Applikationsform ausgewichen werden.

In der EP-A-0 272 097 ist erwähnt, daß Progesteron aus der Nasenhöhle in einer Weise absorbiert wird, daß Blutspiegel die Folge sind, die zu intravenöser Applikation nahezu gleichwertig sind.

In der EP-A-0 160 501 ist eine intranasale Formulierung für Catecholamin offenbart, das in Fettsäure (oder -ester) suspendiert und mit Polyoxyethylen emulgiert ist.

Aus der nicht-vorveröffentlichten Patentanmeldung P 42 14 053.3 derselben Anmelderin ist ein Dosierspray zur Applikation von Vorläufern des Testosterons bekannt.

FR-M-6033 betrifft ein Kombinationspräparat aus Dopamin, Amobarbital und Vitamin C mit adrenomimetischen und sedativen Wirkungen auf das Nervensystem, unter anderem auch in Form eines Aerosols.

Der vorliegenden Erfindung liegt die Aufgabe zugrunde, eine Darreichungsform zur Verfügung zu stellen, durch die der Zutritt bestimmter Wirkstoffe zum zentralen Nervensystem begünstigt und damit eine Reduktion der Einzeldosis und/oder die Zufuhr von Wirkstoffen ermöglicht wird, die peroral aus den dargelegten Gründen nicht zugeführt werden können.

Erfindungsgemäß wird diese Aufgabe durch die Verwendung mindestens eines Sexualhormons bzw. mindestens einer Vorstufe eines Sexualhormons im Stoffwechsel oder mindestens eines Derivats eines Sexualhormons oder einer Kombination derselben, ausgenommen die Vorläufer von Testosteron, oder von Dopamin, einem Dopaminderivat, NADH, NADPH oder einer Kombination derselben zur Herstellung eines Arzneimittels in Form eines Dosiersprays für pernasale Applikation zur gezielten Dosisreduktion oder zur Erleichterung des Durchtritts durch die Blut-Hirn-Schranke gelöst.

Die bevorzugte Dosierung liegt zwischen 2 und 20 mg Wirkstoff pro Sprühstoß.

Weitere Merkmale und Vorteile der Erfindung ergeben sich aus der nachfolgenden Beschreibung zu einer besonders bevorzugten Ausführungsform.

### Applikation von Testosteron mittels Dosierspray

Die Vorteile der Darreichungsform mittels Dosierspray wurden im Vergleich zur oralen Applikation von Testosteron überprüft.

Die perorale Zufuhr von 100 mg Testosteron mittels Kapsel erhöhte den Blutspiegel von einer Ausgangskonzentration zwischen 32,5 und 37,5 nmol/1 auf Werte zwischen 45 - 50 nmol/1. Das Maximum der Konzentrationen wurde in einem Zeitraum von 30 min nach Applikation erreicht, unterlag also starken individuellen Schwankungen.

Das Profil der im Urin ausgeschiedenen Steroide reagierte sehr stark, es kam z.B. zu Erhöhungen des Quotienten Testosteron/Epitestosteron ausgehend vom Normalbereich zwischen 0,9 und 2,8 auf Werte bis zu 60.

Die Kinetik des Blutspiegels verlief individuell sehr unterschiedlich, z.T. wurden schon 60 bis 90 min nach Applikation wieder die Ausgangswerte erreicht.

Bei Applikation des Testosterons mittels Nasenspray wurde zur Erreichung eines gleichen Anstiegs im Blut nur eine Dosis von 7 bzw. 14 mg pro Applikation benötigt.

Die Applikation erwies sich als wesentlich besser steuerbar. Die Anstiege der Blutkonzentrationen betrugen bei verschiedenen Probanden stabil das 1,5fach bis 2fache der individuellen Ausgangskonzentrationen. Der Zeitraum, in dem die maximale Blutkonzentration erreicht wurde, verkürzte sich deutlich auf 15 bis höchstens 90 min.

Die Störungen im Steroidprofil des Urins waren deutlich geringer, der Quotient Testosteron/Epitestosteron stieg lediglich auf Werte zwischen 15 und 20 an und normalisierte sich innerhalb von 24 Stunden.

Alle Probanden berichteten nach pernasaler Applikation übereinstimmend von einer verbesserten psychophysiologischen Belastbarkeit. Dieser Effekt war bei peroraler Applikation nicht beobachtet worden. Er wird auf die bevorzugte Beeinflussung von zentralnervalen Steroidrezeptoren zurückgeführt, die vermutlich durch eine Erleichterung des Durchtrittes durch die Blut-Hirn-Schranke bei pernasaler Applikation verursacht wird.

### Applikation von Antiparkinsonmitteln

Für Therapeutika, die zur Behandlung des M. Parkinson eingesetzt werden, ist der Durchtritt durch die Blut-Hirn-Schranke wesentliche Wirkungsvoraussetzung.

Aufbauend auf die vorstehend dargelegten Ergebnisse wurde in einer Pilotstudie NADH pernasal appliziert. In Pilotstudien mit parenteraler Applikation von 5 - 10 mg NADH (Infusion über 30 min) war gefunden worden, daß die Frequenz des Tremors deutlich vermindert und die Beweglichkeit sowie die Zielgenauigkeit der Bewegungen verbessert wird. Die intranasale Applikation von NADH zeigte bei gleicher Dosierung die gleichen Wirkungen. Damit besteht die Möglichkeit, die risikobehaftete und belastende intravenöse Infusion zu vermeiden und stattdessen über den Dosierspray eine wesentlich bessere alters- und krankheitsgerechte Applikation zu praktizieren.

Bei pernasaler Applikation kann darüber hinaus der Wirkstoff in Pulverform vorliegen. Durch den Sprühstoß erfolgt eine sehr feine Verteilung im Bereich der Nase und Nasennebenhöhlen, die die Resorption über eine große Fläche begünstigt. Die galenische Formulierung als Trockensubstanz beseitigt die Probleme mit der Haltbarkeit und Stabilität des Wirkstoffes. Die bevorzugte Darreichung als Nasenspray ist damit für die ambulante Therapie des M. Parkinson besonders geeignet.

## Patentansprüche

1. Verwendung mindestens eines Sexualhormons bzw. mindestens einer Vorstufe eines Sexualhormons im Stoffwechsel oder mindestens eines Derivats eines Sexualhormons oder einer Kombination derselben, ausgenommen die Vorläufer von Testosteron, oder von Dopamin, einem Dopaminderivat, NADH, NADPH oder einer Kombination derselben zur Herstellung eines Arzneimittels in Form eines Dosiersprays für pernasale Applikation zur gezielten Dosisreduktion oder zur Erleichterung des Durchtritts durch die Blut-Hirn-Schranke.

2. Verwendung nach Anspruch 1, gekennzeichnet durch eine Dosierung zwischen 2 und 20 mg Wirkstoff pro Sprühstoß.

## Claims

1. Use of at least one sex hormone or at least one precursor of a sex hormone in the metabolic process or at least one derivative of a sex hormone or of a combination thereof, apart from the precursors of testosterone or of dopamine, a dopamine derivative, NADH, NADPH or a combination thereof for producing a medicament in the form of a dispensing spray for pernasal application for controlled dosage reduction or for facilitating the passage through the blood-brain barrier.

2. Use according to claim 1, characterised by a dosage between 2 and 20 mg active principle per spray burst.

## Revendications

1. Utilisation d'au moins une hormone sexuelle respectivement d'au moins un étage préliminaire d'une hormone sexuelle, lors d'un échange de matière ou au moins d'un dérivé d'une hormone sexuelle ou d'une combinaison de ceux-ci, à l'exception des précurseurs de la testostérone, ou de la dopamine, d'un dérivé de dopamine, du NADH, du NADPH ou d'une combinaison de ceux-ci pour la préparation d'un médicament sous forme d'une pulvérisation dosée destinée à une application pernasale pour une réduction dosée à dessein ou bien pour faciliter le passage à travers la barrière sanguine-cérébrale.

2. Utilisation selon la revendication 1, caractérisée par un dosage compris entre 2 et 20 mg de substance active, par prise par pulvérisation.
